# EUROPEAN PATENT SPECIFICATION

(11) **EP 2 576 779 B1**
(45) Date of publication and mention of the grant of the patent: **09.08.2017**
(21) Application number: 11726377.2
(22) Date of filing: 01.06.2011
(51) Int. Cl.: C12N 15/10

(54) **Method for isolating and/or purifying nucleic acid(s)**
Verfahren zum Isolieren und/oder Reinigen von Nukleinsäure(n)
Procédé d'isolation et/ou de purification des acides nucléiques

(30) Priority: 01.06.2010 EP 10005664
(43) Date of publication of application: 10.04.2013
(73) Proprietor: Qiagen GmbH, 40724 Hilden (DE)
(72) Inventor: FABIS, Roland, 40724 Hilden (DE); PETZEL, Jan, 40724 Hilden (DE)
(74) Representative: f & e patent
(86) International application number: PCT/EP2011/059163
(87) International publication number: WO 2011/151428

(56) References cited:
- EP-A2- 0 707 077
- WO-A1-2004/003200
- WO-A1-2010/072821
- WO-A2-02/48164
- WO-A2-2008/097342
- MARTON L J ET AL: "POLYAMINE-DNA INTERACTIONS POSSIBLE SITE OF NEW CANCER CHEMOTHERAPEUTIC INTERVENTION", PHARMACEUTICAL RESEARCH (NEW YORK), vol. 3, no. 6, 1986, pages 311-317, XP007914671, ISSN: 0724-8741
- AHSAN U KHAN ET AL: "Spermine and spermidine protection of plasmid DNA against single-strand breaks induced by singlet oxygen", PROCEEDINGS OF THE NATIONAL ACADEMY OF SCIENCES OF THE UNITED STATES (PNAS), NATIONAL ACADEMY OF SCIENCE, US, vol. 89, 1 December 1992 (1992-12-01), pages 11428-11430, XP007914689, ISSN: 0027-8424
- OH TAE JEONG ET AL: "Polyamines protect against DNA strand breaks and aid cell survival against irradiation in Escherichia coli", BIOTECHNOLOGY TECHNIQUES, vol. 12, no. 10, October 1998 (1998-10), pages 755-758, XP002599273, ISSN: 0951-208X
- LUCIANO D AGOSTINO ET AL: "Nuclear Aggregates of Polyamines", IUBMB LIFE, TAYLOR AND FRANCIS, LONDON, GB LNKD- DOI:10.1080/15216540600662525, vol. 58, no. 2, 1 February 2006 (2006-02-01), pages 75-82, XP007914693, ISSN: 1521-6543 [retrieved on 2008-01-03]

## Description

The present invention relates to a method for isolating and/or purifying one or more nucleic acid(s) from a sample, comprising the steps of essentially separating the nucleic acid(s) from the sample by binding them to a solid phase by means of a non-chaotropic water-soluble binding ligand at a first pH (pH I), and essentially eluting the nucleic acid(s) from the solid phase at a second pH (pH II). The invention further relates to the use of a non-chaotropic water-soluble cationic compound for protecting nucleic acid(s) from enzymatic and/or non-enzymatic degradation and/or decomposition, cleavage, fragmentation and/or unintended modification as well as to a kit for isolating and/or purifying nucleic acid(s) from a sample and/or for protecting nucleic acid(s).

The analysis of nucleic acids, including ribonucleic acids (RNA) and desoxyribonucleic acids (DNA) is of major importance in biologic, medical and pharmacologic research and diagnostics. For example, examination of the nucleic acids of a cell allows to determine the cell's genetic origin and functional activity. In addition, genetic markers for the detection and/or prediction of diseases and/or genetic mutations can be identified. Furthermore, analysis of RNA and DNA is also useful for identifying pathogenic bacteria, fungi and viruses.

However, most of these methods for analysing nucleic acids require nucleic acids as a substrate which are essentially isolated and purified from any other cellular components and further contaminants entrained in the sample in preceding steps, for example during a lysing step. Accordingly, high throughput methods for the isolation and/or purification of nucleic acids are desirable, which allow a quick and reliable isolation and/or purification of the nucleic acids, preferably in an environmentally friendly manner.

Existing methods for the isolation and/or purification of nucleic acids from a sample include the use of phenol/chloroform, methods of salting out, and various solid phase techniques, including the use of gel filtration, ion exchange or affinity resins. In particular binding of nucleic acids to silica matrices or membranes in the presence of chaotropic salts has found wide-spread application. Each of the methods mentioned above has its particular drawbacks, for example the use of flammable, toxic and/or corrosive substances or the need for laborious multi-step procedures, which cannot be automated. Other known methods require the use of particularly adapted solid phases, which in turn cannot be applied to a broad range of nucleic acids of different type, size and/or origin.

Another approach is the use of cationic polymeric compounds to precipitate nucleic acids from a sample solution, such as for example polyethylene imine. The complex formed by the nucleic acids and the polymer is separated from the remaining sample solution by filtration or centrifugation and the nucleic acids are then released from the complex. However, harsh conditions are often required for releasing the nucleic acids from said complex.

EP 0 707 077 describes weakly basic water-soluble polymers and their use for precipitating nucleic acids, present for example in a lysate, at an acidic pH. However, release of the complexed nucleic acids requires extreme conditions in view of pH, temperature and/or high salt concentrations, which may lead to denaturation and/or degradation of the nucleic acids, in particular of RNA. In addition, further steps of purification are often required prior to storage, analysis and/or amplification of the nucleic acids.

WO 2004/003200 and WO 2006/083962 also describe the use of polymers comprising quaternary amino groups for precipitating nucleic acids (in particular DNA) from solution. Again, a high salt concentration is necessary to re-dissolve the DNA from the complex. In consequence, the DNA solution obtained from these methods cannot be directly used in downstream applications. Furthermore, in particular for automated high throughput applications the use of a solid phase for separating the nucleic acids from the remaining sample solution often is more desirable than a precipitation approach.

The so called "charge-switch"-technique makes use of a solid phase able to bind nucleic acids at a first pH, at which the surface of the solid phase is positively charged. In consequence it can bind nucleic acids which due to their phosphate backbone have a negative net charge. After optional washing steps, the nucleic acids are eluted from the charge-switch solid phase by rinsing said phase with an elution buffer at a second pH, wherein said second pH is above the first pH and the pKₐ-value of the solid phase. At said second pH the former positive charge of the solid phase is neutralized or even inverted, thus minimizing the attracting interactions between the solid phase and the nucleic acids.

WO 02/48164 and related patents by the same inventors describe a method for extracting nucleic acids from a sample using various charge-switch materials. These charge-switch materials are obtained by modifying a commercially available solid phase, such as for example magnetic beads, polystyrene beads or multiwell plates with ionisable groups, preferable by covalently binding substances comprising these ionisable groups to the solid phase using a chemically coupling agent such as for example the carbodiimide EDC.

Covalently modified solid phases and their use in methods for isolating and/or purifying nucleic acids using a charge-switch-technique are described for example in US 2008/0118972, too.

Charge-switch materials often allow binding and releasing of the nucleic acids under rather mild conditions. On the other hand, however, they do require a highly modified polymer and/or surface of a solid phase in order to guarantee an adequate affinity of the nucleic acids for the solid phase. In addition, this affinity varies depending upon the type, size and origin of the nucleic acids. In consequence, the user either has to buy and store a plurality of different expensive charge-switch materials, or he has to cope with a rather poor purity and/or yield in some cases, which in turn may require additional steps of concentrating and/or purifying.

Accordingly, the object of the present invention was to provide a method for isolating and/or purifying nucleic acids from a sample by means of a solid phase, which allows binding and releasing of the nucleic acids at mild conditions with respect to pH and salt concentration, i.e. under low salt concentration, which easily can be adapted to nucleic acids of different type, size and origin by the user without the need for buying and storing a plurality of different ready-to-use solid phases. A further object underlying the present invention was to provide a method for isolating and/or purifying nucleic acids which does not require a pre-treatment of the solid phase.

This object is met by the method of the present invention. The present invention provides a method for isolating and/or purifying one or more nucleic acid(s) from a sample, comprising the steps of:
1) separating the nucleic acid(s) from the sample by binding the nucleic acid(s) to a solid phase by means of a binding ligand at a first pH (pH I), and
2) eluting the nucleic acid(s) from the solid phase at a second pH (pH II),
wherein the binding ligand is a non-chaotropic water-soluble cationic compound comprising at least one basic moiety and/or at least one quaternary ammonium moiety, wherein said binding ligand forms a complex with the nucleic acid(s) at a pH below or equal to pH I and wherein the solid phase and the binding ligand are brought into contact upon or after contacting the nucleic acid(s) with the binding ligand, but not before, and wherein said first pH is lower than said second pH (pH I < pH II) and said second pH is lower than the logarithmic acidity constant pKa of the conjugated acid of said basic moiety of the binding ligand (pH II < pKa), if the binding ligand comprises one or more basic moieties or said second pH is above seven (pH 7 < pH II), if the binding ligand comprises quaternary ammonium groups, or if the binding ligand comprises both, at least one basic moiety and at least one quarternary ammonium moiety, said second pH is below the pKa of the at least one basic moiety but above pH 7.

It has surprisingly been found that non-chaotropic water-soluble cationic compounds comprising at least one basic moiety and/or at least one quaternary ammonium moiety are able to form a complex with the nucleic acid at a first pH (pH I) within a first pH range and that these complexes can be bound in an essentially quantitative manner to a solid phase at said pH I. After optional washing steps, the nucleic acids can be eluted from the solid phase within a second pH range (pH II). Thus, following the method of the present invention, it is not necessary to modify a commercially available solid phase prior to the purification step itself or to buy an expensive modified solid phase. Instead, a standard solid phase and a binding ligand are simply brought into contact upon contacting or after having contacted the nucleic acids with the binding ligand. The binding ligand and the nucleic acids may be mixed before contacting the resulting mixture with a solid phase, or the nucleic acids, the binding ligand and the solid phase may be brought into contact/mixed simultaneously. Surprisingly, the formation of a complex or aggregation of the cationic compound and the nucleic acid before getting into contact with the solid phase does not prevent a binding of the cationic compound within the complex to said solid phase. Due to this unexpected effect, there is no longer a necessity to pre-treat the solid phase in order to bind the binding ligand thereto. Thereby a facilitation and acceleration of the isolation procedure may be achieved while at the same time still obtaining high yields.

Upon contacting the sample comprising one or more nucleic acid(s) with the water-soluble cationic compound (the binding ligand), a complex is formed comprising nucleic acid(s) and cationic compounds due to the negatively charged phosphate backbone of the nucleic acid(s). The complex may precipitate from the solution, either completely or in part, or may stay in solution as well, depending upon the cationic compound, its concentration, the dissolving medium (e.g. pH-value, temperature or salt concentration), and the type, size and origin of the nucleic acids. For the method of the present invention, however, it is not important whether or not the complex precipitates from the sample solution, since the extent of precipitation does not influence the extent and strength of the complex' binding to the solid phase.

A further advantage of the method of the present invention is the fact that the complex formed by the nucleic acid and the binding ligand protects the nucleic acid(s) from enzymatic and/or non-enzymatic degradation and/or decomposition, cleavage, fragmentation and/or unintended modification. This protective effect lasts from the moment of adding the binding ligand to the sample comprising the nucleic acid(s) to the point of eluting of the nucleic acid(s) from the solid phase, as the nucleic acid(s) remain complexed to the binding ligand during almost the whole isolating and/or purification method of the present invention, i.e. until the step of eluting it/them from the solid phase.

In addition, the method of the present invention is quick and easy-to-handle and can be automated for high throughput applications, for example by using a magnetic solid phase. Both binding and eluting conditions are mild and take place at a moderate pH under a low salt concentration, which might for example be as low as 50 mM, based on the solution obtained after mixing the sample with the binding agent or the eluate obtained, respectively. For example, efficient binding may be effected at a pH of about pH 3-7, preferably of about pH 5 - 6, whereas efficient elution of the nucleic acids from the solid phase may for example be achieved using elution buffers having a pH of about 7.5 to 9.0, preferably of about 8.5. In both, the binding and the eluting buffer, the salt concentration may for example preferably be less than 1 M, more preferably less than 0.5 M and even more preferably less than 0.1 M. It may also be preferred to use a completely salt-free buffer, in particular in the binding step. The nucleic acids isolated and/or purified according to the method of the present invention thus can be directly used in various downstream applications such as enzymatic nucleic acid amplification and modification reactions in general, including PCR, restriction digest, transfection, and short tandem repeat (STR) analyses, without being limited to these. In addition, no toxic and/or flammable chemicals are required in the method of the present invention. Both, RNA and DNA, can be selectively isolated using the method of the present invention.

In addition, no time-consuming and/or expensive chemical modification of a solid phase is necessary prior to the isolation and/or purification procedure itself, and both, the solid phase as well as the binding ligand, can be easily adapted or selected to a particular type of nucleic acid to be isolated. Nevertheless, for many different nucleic acids high binding capacities and a high purity of the nucleic acid isolated by the method of the present invention even are observed when using a kind of standard conditions not adapted to a particular type of nucleic acids.

Nucleic acid(s) which can be isolated and/or purified using the method of the present invention include DNA and RNA, in particular genomic DNA (gDNA), plasmid DNA, PCR-fragments, cDNA, rRNA, miRNA, siRNA as well as oligonucleotides and modified nucleic acids such as so-called peptide or locked nucleic acids, respectively, (PNA or LNA), of microbial, including viral, bacterial and fungi, human, animal or plant origin. In addition, also hybrids formed of DNA and RNA can be purified, without being limited to these.

The sample to be processed by the method of the present invention may represent any sample comprising nucleic acids, and preferably is a biological sample, either in its natural state or in a processed form. Preferably the samples may include body fluids such as blood, serum, sputum, faeces, plasma, sperm, cerebro-spinal fluids, saliva, etc., human, animal or plant tissues and tissue cultures, microbial human, animal or plant cells and cell cultures, and human or animal organs or parts thereof, such as for example liver, kidney or lung. In addition, the samples may represent swabs or PapSmears, stabilized biologic samples as PreServCyt (Becton Dickson, New Jersey, USA) or Surepath (Cytyc, Massachusetts, USA) or fluid samples such as waste or drinking water, juices, or food without being limited to these. In addition, the sample may represent a processed biologic sample such as for example a human, animal or plant cell lysate, bacterial lysate, a paraffin embedded tissue, aqueous or buffered solutions of a sample, or gels.

If the nucleic acid(s) to be isolated is/are present in a cellular material, it may be preferred to first destroy the cellular material according to any method known from the state of the art for releasing the nucleic acid(s) from a cell, such as for example by lysing, before further processing them according to the method of the present invention.

In addition, the sample may also comprise nucleic acids stabilizing reagents, such as for example RNAlater, RNA Protect, PAXgene System, Allprotect Tissue reagent (all available from QIAGEN, Hilden, Germany) or cationic surfactants like, for example, tetradecyltrimethylammonium oxalate (also known under the trademark name Catrimox-14) etc.

The first pH (pH I), is in a pH range in which the nucleic acids present in the sample are bound to the solid phase by means of the binding ligand. The second pH (pH II) is in a pH range at which the nucleic acid(s) are eluted from the solid phase. Said first pH preferably may be lower than the second pH (pH II) (pH I < pH II). The binding ligand preferably may represent an organic substance, preferably an organic substance as described in detail in the following. If the binding ligand comprises one or more basic moieties, said second pH is lower than the logarithmic acidity constant pKₐ of the conjugated acid of said basic moiety of the binding ligand (pH II < pKₐ).

If the binding ligand comprises more than one basic moiety per molecule, said second pH is lower than the logarithmic acidity constant of the weakest basic moiety (pH II < pKa), provided that said moiety nevertheless still is a basic moiety, i.e its conjugated acid has a pKₐ above 7 (pKₐ>7).
If on the other hand, the binding ligand comprises quaternary ammonium moieties, said second pH preferably is above 7 (pH 7 < pH II).

If the binding ligand comprises both, at least one basic moiety and at least one quarternary ammonium moiety, said second pH preferably may be below the pKa of the at least one basic moiety. More preferably said second pH may be below the pKa of the at least one basic moiety, but above 7 (pH 7 < pH II < pKa).

The binding ligand is water-soluble and may preferably be added to the nucleic acid in the form of an aqueous solution. Said aqueous solution may comprise further components such as buffering substances, for instance, MES, MEPES, TRIS, Bis-TRIS, phosphates, borates or carbonates, organic solvents, for instance, acetone or acetonitrile, carbohydrates, polyethyleneglycols, polyols, small amounts of organic or inorganic salts, but preferably does not comprise any chaotropic substance or alcohol. If the binding ligand comprises at least one basic moiety, the logarithmic acidity constant pKₐ may range from 9 to 12, preferably from 10 to 12. The pKa preferably may be in this range even if the binding ligand comprises a mixture of basic moieties and quarternary ammonium groups.

In terms of the present invention, a basic moiety refers to a functional group which is able to accept hydrogen ions, i.e. which can be protonated at a pH value which is below the pKa value of said moiety.

The binding ligand also may comprise at least one or at least two quaternary ammonium moieties. In a particular preferred embodiment, the binding ligand may comprise more than three quaternary ammonium moieties.

In terms of the present invention a quaternary ammonium moiety is a functional group comprising a positively charged nitrogen atom bound to four carbon atoms, for example carbon atoms of alkyl chains.

It also may be preferred that a binding ligand comprises both at least one basic moiety and at least one quaternary ammonium moiety in any ratio. The binding ligand may for example represent a block polymer, comprising blocks of quaternary ammonium moieties and blocks of basic moieties, for example amino moieties, such as, for instance, poly(dimethylamine-co-epichlorohydrin-co-ethylenediamine).

It is also possible to use a combination of two or even more different binding ligands as defined according to the present invention in any ratio.

The basic functional moieties of the binding ligand of the present invention may also represent moieties used as anion exchanging groups in anionic exchange materials.

Preferably, the binding ligand may comprise more than one, preferably more than two and most preferably more than three basic moieties per molecule and said basic moieties preferably may represent primary, secondary, and/or tertiary amino groups.

The binding ligand preferably can be a primary, secondary, or tertiary mono- or polyamine. These compounds may be further substituted by alkyl, alkenyl, alkinyl or aryl groups, without being limited to these. In addition, one or more carbon atoms in the ring or chain may be substituted by hetero atoms such as oxygen, nitrogen, sulphur or silicon, without being limited to these. They may be linear, branched or in a cyclic form, including cyclic alkylamines and aromatic amines. Preferably, the binding ligand may be selected from the group comprising linear and branched alkylamines, cyclic amines, aromatic amines, heteroaromatic amines and polyamines of the general structure R¹R²N[(CH₂)ₓ₁₋ₓ₇NR³]_{y}R⁴, wherein R¹, R², and R³, and R⁴ independently are selected from the group comprising hydrogen, and linear or branched C₁-C₁₈ alkyl groups, including methyl, ethyl,
n-propyl, isopropyl, n-butyl, *sec*-butyl, and *tert*-butyl. x1-x7 independently represent an integer from 2 to 8, i.e. 2, 3, 4, 5, 6, 7 or 8 and y ranges of from 1 to 7, i.e. 1, 2,3,4, 5, 6, or 7. If y is greater than 1, the binding ligand comprises more than one aminoalkyl group [(CH₂)ₓ₁₋ₓ₇NR³], and R³ in each of these groups may be the same or different (R^{3I}-R^{3VII}). This general structure includes particularly amines the following general formulae

R¹R²N(CH₂)ₙNR³R⁴

R¹R²N(CH₂)ₙNR³(CH₂)ₘNR⁴R⁵

R¹R²N(CH₂)ₙNR³(CH₂)ₘNR⁴ (CH₂)ₒNR⁵R⁶

R¹R²N(CH₂)ₙNR³(CH₂)ₘNR⁴(CH₂)ₒNR⁵(CH₂)ₚNR⁶R⁷

R¹R²N(CH₂)ₙNR³(CH₂)ₘNR⁴(CH₂)ₒNR⁵(CH₂)ₚNR⁶ (CH₂)_{q}NR⁷R⁸

R¹R²N(CH₂)ₙNR³(CH₂)ₘNR⁴(CH₂)ₒNR⁵(CH₂)ₚNR⁶(CH₂)_{q}NR⁷(CH₂)ᵣNR⁸R⁹

R¹R²N(CH₂)ₙNR³(CH₂)ₘNR⁴(CH₂)ₒNR⁵(CH₂)ₚNR⁶(CH₂)_{q}NR⁷(CH₂)ᵣNR⁸(CH₂)ₛNR⁹R¹⁰

wherein R¹ to R¹⁰ each can be a group as defined for R¹ to R⁴ above, and n, m, o, p, q, r, and s independently represent an integer from 2 to 8. Examples include e.g. *N*-propyl-1,3-propanediamine (R¹ = C₃H₇, R²-R⁴ = H and n = 2), spermidine (R¹-R⁵ = H, n = 3, m = 4), spermine (R¹-R⁶ = H, n = 3, m = 4, o = 3) or pentaethylene hexamine, R¹-R⁸ = H, n = m = o = p = q = 2). A particularly preferred binding ligand of this group is spermine.

Further preferred binding ligands of this type comprise cadaverine (1,5-diaminopentane), putrescine (1,4-diaminobutane) and tetraethylene pentamine.

Preferably, the amino groups in the binding ligand are not adjacent to an electron-withdrawing group, such as for example a carboxyl group or a carbonyl group, a C=C-double bond or a β-hydroxyethyl group, in order to ensure that the pKₐ-value is between 9 and 12. In terms of the present invention a moiety is adjacent to an electron-withdrawing group or a C=C double bond or a β-hydroxyethyl group, if said moiety and the electron-withdrawing group or the C=C double bond or the β-hydroxyethyl group are separated by 3, 2 or less carbon atoms.

Nevertheless, even though compounds, wherein an aminogroup is adjacent to an electron-withdrawing group, a C=C-double bond or a β-hydroxyethyl group usually are not suitable as a binding ligand in terms of the present invention, the additional presence of such compounds in the sample, binding solution or the like usually does not negatively influence the binding between the nucleic acid and the solid phase which is mediated by a binding ligand. If, for instance, a binding ligand such as polyethylene imine (PEI) is present, the presence of MES does not negatively influence the binding to a solid phase. In the absence of a binding ligand, however, nucleic acids are not bound to the solid phase in the presence of MES (at least not, if MES is present in a concentration of about up to 50 mM), at a pH in the preferred range for binding according to the present invention (see example 4).

The binding ligand also may be selected from the group comprising polylysine (D- or L-polylysine as well as mixed D- and L-polylysines), polyarginine (D- or L-polyarginine as well as mixed D- and L-polyarginines), protamines, linear and branched polyethylene imines, polyallylamine hydrochlorides, polyvinylamine hydrochlorides, poly(diallyldimethylamine hydrochlorides) and polydiallylmethylamine hydrochlorides. Said polymeric binding ligands may be linear or branched and furthermore functionalized, for instance, alkoxylated, e.g. ethoxylated, and/or end-capped.

The molecular weight M_{w} or the average molecular weight Mₙ, respectively, of said polymers is not particularly limited and may, for example, range of from 300-100,000 (g/mol in the case of M_{w}). Preferably, polymers having an (average) molecular weight of about 1,000 (g/mol) or less may be used.

The binding ligand may also comprise a plurality of quaternary ammonium moieties, i.e. it may represent a polyammonium compound such as for example polydiallyl dimethylammonium chloride, poly(diallyl methylammonium chloride), polydimethylamine-co-epichlorohydrine-co-ethylene diamines or poly(acrylamide-co-diallyldimethylammonium chloride).
In a preferred embodiment carboxy-functionalized magnetic beads and spermin as a binding ligand is used. In another preferred embodiment carboxy-functionalized magnetic beads and polyethylene imine as a binding ligand is used.
In another preferred embodiment carboxy-functionalized magnetic beads and pentaethylene hexamine as a binding ligand is used
In another preferred embodiment carboxy-functionalized magnetic beads and polydiallyl dimethylammonium chlorid as a binding ligand is used.

For complex formation between the nucleic acid(s) and the binding ligand it is essential that the binding ligand is in a cationic form. Thus, if the binding ligand comprises basic moieties, it is important that at least some of these basic moieties are protonated. If the pKₐ of the basic moieties is in the preferred range of from 9 to 12, a pH of equal to or less than 7.0 might be enough to ensure that at least some of these basic groups are present in their protonated, i.e. cationic form. The binding ligand may be protonated before contacting it with the nucleic acids. It is also possible to first mix the unprotonated binding ligand and the nucleic acid(s) and then lower the pH until the binding ligand gets protonated. A pH of from 1 to 8, in particular of from 1 to 7 usually ensures at least partial protonation of the binding ligand. For increasing the amount of protonated binding ligands having primary, secondary, or tertiary amino groups (and optionally the degree of protonation within one binding ligand comprising more than one of these amino groups), it is preferred to allow complex formation taking place at a pH ranging of from 1 to 7, preferably of from 3 to 6, more preferably of from 5 to 6, including e.g. pH 5.2, 5.4, 5.6 or 5.8.

If the binding ligand comprises quaternary alkyl ammonium moieties, no protonation is necessary, as these moieties are already present in a cationic form. Nevertheless, a (slightly) acidic pH like e.g. of from pH 5 to below pH 7, preferably of from 5 to 6 might be favorable for complex formation in these cases as well.

The solid phase of the present invention preferably may comprise a carrier material, which preferably may be selected from the group comprising organic polymers, polysaccharides, and inorganic carriers, more preferably from the group comprising polystyrenes, polyacrylates, polymethacrylates, polyurethanes, nylon, polyethylene, polypropylene, polybutylidene, and their copolymers, agarose, cellulose, dextrane, or the commercially available gel filtration media sephadex and sephacryl, chitosane, silica, quartz, glass, metal oxides, and metal surfaces.

The use of a solid phase facilitates separation of the nucleic acid from the remaining parts of the sample as well as automatization of the method. The solid phase of the present invention is not particularly limited to a special form and may be in the form of for example (a) particle(s), including magnetic particles, (a) bead(s), including magnetic beads, a surface coating, a tube, a paper, a multiwell plate, a chip, a microarray, a tip, a dipstick, a rod, a filter plug or pad, a resin, including resins for column and spin chromatography, a mesh, and/or a membrane.

Magnetic particles are easy to handle, in particular in view of an automated separation of the particles from a sample solution. Thus, the solid phase used in the method of the present invention preferably may be magnetic, including paramagnetic, ferrimagnetic, ferromagnetic or superparamagnetic materials. Most preferably the particles may be ferrimagnetic or superparamagnetic beads or particles.

At a pH below 7 the surface of the solid phase of the present invention preferably shall be neutral or only slightly negatively charged. An easy-to-handle method of determining the surface charge of a solid phase, which is also suitable for small-sized beads and/or particles, is to measure the so-called zeta potential. It may, for example, be preferred that at a pH in the range of about 4 to 5 the surface used in the method and/or comprised in the kit of the present invention has a zeta potential of from 0 to -40 mV, preferably of from 0 to -30 mV. On the other hand, the surface clearly should be charged negatively at a pH above 7, i.e. the zeta potential should, for example, be at least -10 mV lower at a pH above 7 than at a pH of 4, more preferably at least-15 mV and most preferably at least-20 mV. The values given herein are the standard zeta potential SOP determined using a Zetasizer Nano ZS (Malvern Instruments GmbH, Herrenberg, Germany) and a green disposable zeta cell DTS1060.

Thus, the solid phase preferably may comprise acidic moieties on at least a part of those surfaces which come into contact with the sample and/or the binding ligand. Said acidic moieties preferably may be selected from the group comprising carboxylic (-CO₂H), sulphonic (-SO₃H), phosphinic (-PO₂H), and phosphonic (-PO₃H) groups. The acidic moieties may also represent acidic hydroxyl groups including phenolic hydroxyl groups, hydroxyl groups present at silica surfaces (silanol groups) or metal oxide species, including natural occurring minerals such as hydroxyapatite, comprising surface-exposed hydroxyl groups M-OH, wherein M may, for example, represent aluminum, calcium, titanium, zirconium, iron, or mixtures thereof, without being limited to these. Carboxylic groups may be particularly preferred. In an acidic medium, these carboxylic groups are protonated to a large (although not necessarily complete) extent. Thus, a surface functionalized with carboxylic groups is almost neutral or only slightly negatively charged at an acidic pH. In an alkaline medium on the other hand, a high percentage of the carboxylic groups is deprotonated and thus is present as an anionic carboxylate ions. Accordingly, in an alkaline medium a surface functionalized with carboxylic groups is negatively charged. The typical pKₐ-value of aliphatic carboxyl groups is in the range of from 3 to 5. Thus, a surface functionalized with aliphatically bound carboxyl groups will be present in a predominantly protonated state at the preferred binding conditions of the present invention. However, since an equilibrium exists between the protonated and the deprotonated form, a small amount of carboxyl moieties still is present in the carboxylate form at the preferred binding conditions, e.g. at a pH about 4 - 6. This small amount is sufficient to attract and bind the complex formed by the nucleic acid and the binding ligand whose net charge is (slightly) positive.

It has surprisingly been found that the number of acidic moieties on the surface of the solid phase is not of major importance for the amount of complex which can be bound to the solid phase and may be chosen from a wide range. For instance, the carboxy loading, i.e. the number of accessable/available carboxyl groups may for example range from 1 to 1.000 µmol per g of the solid phase, preferably of from 10 to 600 µmol/g. For any other acidic groups the same ratio may be suitable.

The ratio of binding ligand to nucleic acid preferably may be equal to or above 0.5 : 1 (w/w), preferably in the range of from 0.5 : 1 to 10 : 1, more preferably in the range of from 0.5 : 1 to 2 : 1, and most preferably may be1:1.

In a sample comprising an unknown amount of nucleic acids, the amount may be, for example, determined photometrically, which is well known to a person skilled in the art.

The acidic groups may be attached directly to the surface of the solid phase or may be a part of it. The acidic groups also may be attached to the solid phase using an adequate spacer or linker, a plurality of which is known in the state of the art, including for example hydrocarbon chains, polyethylene, polyglycols or functionalized silanes, including linear or branched molecules.

After or just upon mixing the binding ligand and the nucleic acid(s) the mixture is brought into contact with said solid phase. The complex formed by the nucleic acids and the binding ligand binds to the solid phase at a pH (pH I; binding pH) which preferably is at least two pH units below the pKₐ of at least one of the basic moieties, i.e. if the pKₐ is 9, pH I is selected to be equal to or below pH 7. If quaternary ammonium compounds are present in the binding ligand, the pH preferably is equal to or below pH 6.0. The step of binding the nucleic acid to the solid phase according to step 1) may be carried out at a pH of from 1 to 8, preferably of from 2 to 7, and more preferably of from 3 to 6, particularly preferably of from 4 to 5, including pH 4.5. Thus, binding can be achieved at slightly acidic, i.e. very mild conditions which improves the quality of the nucleic acids obtained and prevents their decomposition and/or degradation. It is further preferred that the salt concentration in the sample is less than 1 M, preferably less than 0.5 M, more preferably less than 0.25 M, and most preferably less than 0.1 M.

After binding the complex to the solid phase, the solid phase may be washed at least once using an aqueous washing liquid / washing buffer comprising a salt concentration of less than 0.5 M, preferably of less than 0.2 M, more preferably of less than 0.1 M, even more preferably of less than 50 mM, and most preferably of less than 25 mM. Pure water, preferably nuclease-free water, may be used for washing as well. In addition, the aqueous solution may comprise organic solvents miscible with water such as alcohols, polyols, polyethylene glycols, acetonitrile or further water-soluble organic compounds such as carbohydrates for example.

As washing liquids aqueous alcoholic solutions generally may be used, in particular aqueous solutions comprising 10 - 80 % (v/v) alcohol, i.e. including e.g. 10, 15, 20, 25, 30, 35, 40, 45, 50, 55, 60, 65, 70, 75, or 80% of an alcohol, preferably of ethanol or isopropanol. However, in a preferred embodiment the washing liquid is an aqueous solution that does not contain any alcohol, in particular it does not contain ethanol and/or propanol, especially not isopropanol. The concentration of alcohol in the washing liquid, therefore, preferably is 5 % (v/v) or lower, more preferred 3 % (v/v) or lower, even more preferred 1 % (v/v) or lower, more preferred 0,5 % or lower and most preferred 0 % (v/v). Surprisingly it has been found that pure water represents a suitable washing liquid in the present method. Therefore, the newly developed system allows to work under solvent free conditions if desired, i.e. there is a reduced exposure of the lab personnel as well as the environment by hazardous substances.

After this/these optional washing step(s), the step of eluting the nucleic acid(s) from the solid phase is carried out at a pH (pH II) which is above the pH at which binding took place (pH I). In consequence, the basic moieties of the binding ligand are positively charged to a much smaller extent than they were at the moment of binding, which promotes releasing of the nucleic acids from the complex. In addition, the acidic groups present on (at least a part of) the surface of the solid phase are deprotonated to a grater extent, which in consequence leads to a negative surface charge on said surface. This in turn leads to a repulsion between the nucleic acids and the surface, both of which now have a negative net charge, which further promotes the elution of the nucleic acids. At least the second effect also is observed when binding ligands only comprising quaternary ammonium moieties are used.

The pH-value at which elution is effected (pH II) preferably is at least one pH-unit below the pKₐ value of the basic moieties, but above pH I. Thus, elution can be effected under very mild conditions.

The step of eluting the nucleic acids from the solid phase according to step 2) may be carried out at a pH in the range of from 7.5 to 11, preferably of from 7.5 to 10, and most preferably of from 7.5 to 9. The salt concentration in the elution buffer preferably may be less than 1 M, preferably less than 0.5 M, more preferably less than 0.25 M, and most preferably less than 0.1 M.

Salts suitable to be used in the elution liquid are well-known to a person skilled in the art and comprise for example chlorides of alkali and earth alkaline metals, ammonium chloride, salts of mineral acids, acetates, borates, phosphates, and carbonates. In addition or as an alternative, organic buffering substances such as TRIS, Bis-TRIS, MES, CHAPS, HEPES, and the like may be comprised in the elution buffer, without being limited to these. The elution liquid/elution buffer furthermore may comprise substances such as carbohydrates, organic solvents, in particular alcohols, polyethyleneglycols and/or polyols. Preferably, the elution buffer/elution liquid shall not comprise more than 0.5 M of any chaotropic substance, more preferably not more than 0.1 M, even more preferably not more than 0.05 M and most preferably, the elution buffer/elution liquid does not comprise any chaotropic substance at all.

For sensitive down-stream applications, such as enzymatic reactions, e.g. PCR, preferably nitrogen-free elution buffers may be used. If, for instance, it is intended to directly use the nucleic acid purified by the method of the present invention in a subsequent PCR, it may be advantageous to use an elution buffer which does not comprise any nitrogen-containing compounds. Preferably borate and carbonate-based buffers may be used in this case. It also may be preferred to elute the nucleic acids from the solid phase at elevated temperatures, i.e. temperatures above room temperature. In particular, temperatures in the range of from 70 to 95 °C, preferably from 75 to 90 °C may be used. If these elevated temperatures are used, surprisingly no inhibition of a subsequent PCR has been observed, even if the elution buffer comprises nitrogen-containing groups (example 3). Thus, if elevated elution temperatures, preferably being in the range of from 75 to 90 °C are used, the elution buffer may comprise nitrogen-containing compounds, even if it is intended to directly used the eluate in an enzymatic down-stream application such as PCR.

In a particularly preferred embodiment of the method of the present invention, the ratio of binding ligand added to the sample to the nucleic acids present therein may preferably in the range of from 1:1 to 3:1, more preferably at about 2:1. In this embodiment, binding of the nucleic acids to the solid phase may be effected at pH I being in the range of from 4 to 5, while elution preferably may be carried out at pH II being in the range of from 8 to 9, more preferably at about 8.5. Elution furthermore preferably is carried out at temperatures being in the range of from 75 to 90 °C, in particular if the elution buffer comprises nitrogen-containing compounds and the eluate is supposed to be used in sensitive downstream applications such as PCR. Preferably, the elution buffer is a carbonate or a borate-based elution buffer, preferably not comprising any nitrogen-containing compounds, in particular if elution shall be carried out a temperatures below 75 °C.

In the present invention preferably aqueous elution buffers are used which only comprise a small amount of salt and are essentially free from toxic substances. Thus, the eluates obtained are very pure and usually do not contain any substance which may act as an inhibitor or may otherwise interfere in downstream applications such as PCR, restriction digestion, transfection or sequencing. Accordingly, in a preferred embodiment the eluted nucleic acids may be immediately processed further from the eluate, for example in one of the applications mentioned above, without a need for changing the buffer.

Accordingly, the present invention is particularly suitable for applications in the field of molecular biology, molecular diagnostics, forensic chemistry, medicine, drug and food analysis and applied testing, in both manual and automated methods.

The invention further provides the use of a non-chaotropic water-soluble cationic compound comprising at least one basic moiety and/or at least one quaternary ammonium moiety, which forms a complex with nucleic acid(s) at a pH below or equal to pH 8, preferably 7, more preferably 6, and most preferably 5. For protecting the nucleic acids from enzymatic and/or non-enzymatic degradation and/or decomposition, cleavage, fragmentation, and/or unintended modification, preferably in an isolating and/or purifying method, more preferably in the method described above.

Preferably the above non-chaotropic water-soluble cationic compound(s) is/are used as a binding ligand for this purpose.

The invention further provides a kit for isolating and/or purifying nucleic acid(s) from a sample and/or for protecting nucleic acids, preferably as described above, comprising at least
(a) a binding ligand, preferably a binding ligand as described above, and preferably at least one further component selected from the group comprising
(b) a solid phase, preferably a solid phase as described above,
(c) a binding buffer as described above,
(d) an elution buffer as described above, and
(e) instructions for using the kit.

Preferably the kit may comprise at least the binding ligand (component a), and at least two of components b to e, i.e. components a, b, and c; a, b, and d; a, b, and e; a, c, and d; a, c, and e; or a, d, and e. More preferably the kit may comprise at least the binding ligand (component a), and at least three of components b to e, i.e. components a, b, c and d; a, b, c and e; a, b, d, and e; and a, c, d and e. Most preferably, the kit comprises all of the components a to e.

The instruction preferably describes that the binding ligand is only bound to the solid phase upon contacting or after having contacted the binding ligand with the nucleic acid. This means that it preferably describes that the binding ligand is not bound to the solid phase in the absence of the nucleic acid.
Figure 1 shows the effect of an increasing spermine (binding ligand) concentration on the amount of DNA of different size found in the "flow-through" and the eluate, respectively (example 1).
Figures 2A-2C show the effect of different elution buffers components (2A: sodium carbonate, 2B: borate, 2C: sodium hydroxide), pH and salt concentration on the amount of plasmid DNA found in the eluates (example 2).
Figure 3 shows the results of binding plasmid DNA to various commercially available carboxy beads using different binding ligands and a pH of either 5.0 or 6.1 (example 3).
Figure 4 shows a comparison of the extent of DNA binding to carboxy-functionalized Seradyn MGCM Beads in the presence or the absence of a binding ligand, respectively, at a pH of either 5.0 or 5.5 (example 4).
Figures 5A and 5B show the results of purifying plasmid DNA according to the present invention by eluting the DNA at elevated temperature with respect to the isolated amount of DNA (figure 5A) and the purity of its PCR product (figure 5B) (Example 5). In lanes 1-3 the eluates obtained by using a borate-based elution buffer and in lanes 4-6 by using an TRIS-based elution buffer at 60 °C (lanes 1 and 4), 75 °C (lanes 2 and 4), and 90 °C (lanes 3 and 6), respectively, are shown.
Figures 6A and 6B show the impact of the binding ligand on the yield of plasmid DNA (example 6) with respect to the isolated amount of DNA (figure 6A) and the purity of the PCR products obtained from the eluates (figure 6B).
Figures 7A and 7B show the extent of RNA binding to commercially available carboxy-functionalized beads, using pentaethylene hexamine (1) or spermine (2) as a binding ligand (example 7).
Figure 8 shows an formaldehyde gel of an 16S23S rRNA isolated from Jurkat cells using the method of the present invention (example 8).

### Examples

Unless otherwise noted, Sera-Mag® Magnetic Carboxylate-Modified Partides (Thermo Fisher Scientific Waltham, Ma, USA, previously Seradyn Inc., product-ID 2415-2105; also denoted Seradyn MGCM beads) were used as carboxy-functionalized beads.

### Example 1: Purification of various DNA fragments

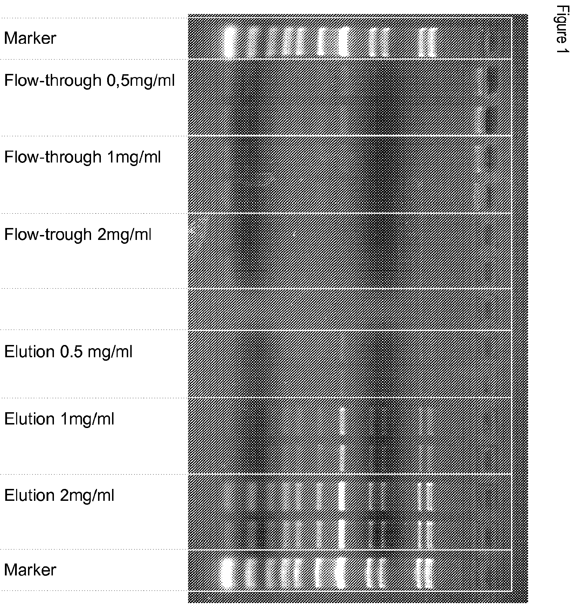

Carboxy-functionalized beads (67 mg beads/mL) were used. The beads were vortexed in their storage buffer and 2.5 µL of the suspension were added into each well of a multiwell plate. After magnetically separating the beads, the storage buffer was removed. 100 µL of a binding buffer comprising 50 mM MES at pH 5.0 were added. 9 µL QIAGEN Gelpilot 1 kb plus ladder (975 ng/well) were mixed with 0.98 µL of aqueous spermine solutions comprising 0.5,1.0 or 2.0 mg/ml of the amine, respectively, at pH of 6.0 by vortexing. 9.98 µL of the mixture comprising 9 µL of the DNA ladder and 0.98 µL of the spermine solution were added to each well, mixed with the beads by manually pipetting up and down and shaking the multiwell plate at 1,000 rpm for 5 min at room temperature on a laboratory shaker. The beads were separated magnetically and the "flow-through" was removed. "Flow-through" refers to the supernatant in the well comprising all the components not bound to the solid phase after the binding step. The beads were washed twice with 100 µL water each, manually resuspended and shaken at 1.000 rpm for 5 min at room temperature. The nucleic acids were eluted from the beads using 20 µL of an elution buffer comprising 50 mM NaCl and 50 mM TRIS (pH 8.5). 20 µL of the eluate and the "flow-through" on an agarose gel were analysed, shown in figure 1.

By adjusting the amount of binding ligand in the binding solution, the amount of DNA in the "flow-through" can be significantly reduced, i.e. an essentially quantitative binding of nucleic acids to the solid phase in the presence of a binding ligand is possible, as can be seen from figure 1.

### Example 2: Purification of plasmid DNA

Seradyn MGCM beads were vortexed and transferred into the wells of a multiwell plate using 1 mg/well. The beads were magnetically separated from the storage buffer and the storage buffer was removed. 100 µL of a binding buffer comprising 50 mM MES (pH 5.0), 5 µL pUC21 (corresponding to 5 µg DNA) as well as 5 µL polyethylene imine solution (c = 1 mg/mL, pH <7) were added to the beads, (ratio polyethylene imine/DNA = 1:1). The mixture was manually mixed by pipetting up and down and shaking the multiwell plate at 1.000 rpm for 5 min at room temperature on a laboratory shaker. The beads were washed twice with 100 µL water each, manually re-suspended and shaken at 1.000 rpm for 5 min at room temperature. The nucleic acids were eluted from the beads using 20 µL of the elution buffers described below and re-suspended manually at 1.000 rpm for 5 min: carbonate buffer (50 mM sodium carbonate, pH 8.5 - 10.0, with 50, 200, 500 or 800 mM NaCl), borate buffer (50 mM borate obtained from a mixture of boric acid and NaOH, pH 8.5 -10.0, with 50, 200, 500 or 800 mM NaCl), 0.1 M NaOH (pH 13 in deionised water) and 0.001 M NaOH (pH 11 in deionised water).

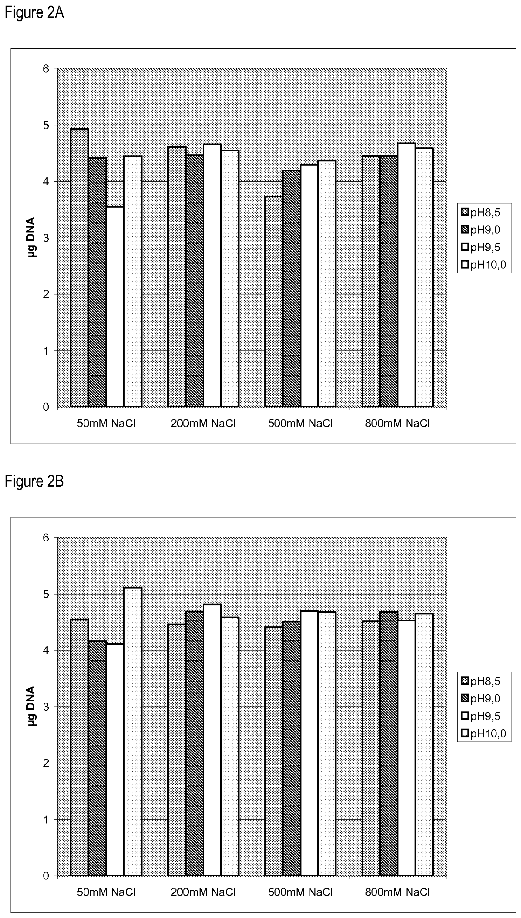

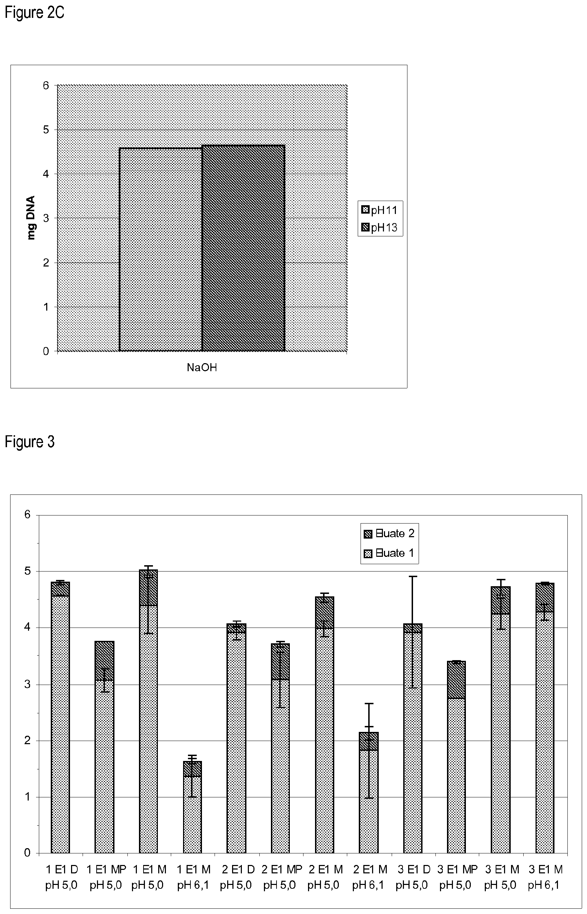

The eluates were analysed at a Nanodrop (Thermo Fisher Scientific Waltham, MA, USA) using a sample size of 2 µL and the respective elution buffers as a blank. The results are presented in figures 2A to 2C. (2A:sodium carbonate, 2B: borate, 2C: sodium hydroxide. As can be seen from a comparison of the results, neither the use of a high pH nor a high salt concentration are necessary in order to obtain a satisfactory yield of DNA in the method of the present invention.

### Example 3: Purification of plasmid DNA using various commercially available beads and different cationic binding ligands

As carboxy-functional beads Dynabeads^{®} M-270 Carboxylic Acid (Invitrogen, Carlsbad, CA, USA; denoted "D" in Fig. 3), Merck MagPrep P-25 Carboxy (Merck, Darmstadt, Germany; denoted "MP" in Fig. 3), and Micromod Micromer-M COOH (Micromod Partikeltechnologie GmbH, Rostock, Germany; denoted "M" in Fig. 3) were used. The beads were vortexed and transferred into different wells of a multiwell plate, each in an amount of 1 mg/well. The beads were magnetically separated from the storage buffer and the storage buffer was removed. 10 µL of a solution comprising DNA and binding ligand were prepared by mixing 5 µg pUC21 (c = 1 µg/µL) and 5 µL of the respective binding ligand (1 E1: polyethylene imine, 2E1: pentaethylene hexamine, 3E1: spermine; each at c = 1 µg/µL in water; pH = 6). 10 µL of the respective solution were added to the beads in each well, respectively. 100 µL of a binding buffer comprising 50 mM MES at a pH of 5.0 or 6.1, respectively, were added to the beads. The mixture was manually mixed by pipetting up and down and shaking the multiwell plate at 1,000 rpm for 5 min at room temperature on a laboratory shaker. The beads were magnetically separated and the "flow-throughs" were collected. The beads were washed with 100 µL of water, manually re-suspended and shaken on a laboratory shaker at 1,000 rpm for 5 min at room temperature. The nucleic acids were eluted from the beads using 50 µL of an elution buffer comprising 50 mMNaCl and 50 mM TRIS at pH 8.5. The eluates of this first elution steps were collected and said elution step was repeated. The eluates obtained from the first and the second elution step, respectively, were analyzed separately at a SpectraMAX plus (Molecular Devices, Sunnyvale, CA, USA)by measuring the absorbance, a wavelength of 260, 280 and 320 nm, respectively, using 100 µL of the elution buffer described above as a blank. For analyzing the obtained eluates 40 µL of the respective eluate was diluted with 60 µL of the elution buffer described above prior to analyzing.

The results are presented in table 1 and figure 3.

| Sample | 260/280 | 280/260 | 260/320 | Average [µg/well] |
|---|---|---|---|---|
| First elution step | | | | |
| 1 E1 Dynal COOH pH 5.0 | 1.859 | 0.538 | 0.732 | 4.57 |
| 1 E1 MagPrep pH 5.0 | 1.887 | 0.530 | 0.513 | 3.07 |
| 1 E1 micromer pH 5.0 | 1.887 | 0.530 | 0.646 | 4.39 |
| 1 E1 micromer pH 6.1 | 1.895 | 0.528 | 0.261 | 1.37 |
| 2 E1 Dynal COOH pH 5.0 | 1.877 | 0.533 | 0.612 | 3.91 |
| 2 E1 MagPrep pH 5.0 | 1.890 | 0.529 | 0.548 | 3.08 |
| 2 E1 micromer pH 5.0 | 1.903 | 0.526 | 0.622 | 3.98 |
| 2 E1 micromer pH 6.1 | 1.896 | 0.527 | 0.387 | 1.83 |
| 3 E1 Dynal COOH pH 5.0 | 1.884 | 0.531 | 0.515 | 3.92 |
| 3 E1 MagPrep pH 5.0 | 1.898 | 0.527 | 0.439 | 2.74 |
| 3 E1 micromer pH 5.0 | 1.906 | 0.525 | 0.647 | 4.24 |
| 3 E1 micromer pH 6.1 | 1.899 | 0.527 | 0.700 | 4.28 |
| | | | | |

| Second elution step | | | | |
|---|---|---|---|---|
| | | | | |
| 1 E2 Dynal COOH pH 5.0 | 1.728 | 0.579 | 0.040 | 0.23 |
| 1 E2 MagPrep pH 5.0 | 1.857 | 0.539 | 0.109 | 0.68 |
| 1 E2 micromer pH 5.0 | 1.746 | 0.573 | 0.109 | 0.62 |
| 1 E2 micromer pH 6.1 | 1.616 | 0.619 | 0.046 | 0.26 |
| 2 E2 Dynal COOH pH 5.0 | 1.270 | 0.787 | 0.019 | 0.16 |
| 2 E2 MagPrep pH 5.0 | 1.838 | 0.544 | 0.105 | 0.63 |
| 2 E2 micromer pH 5.0 | 1.750 | 0.571 | 0.098 | 0.55 |
| 2 E2 micromer pH 6.1 | 1.729 | 0.578 | 0.064 | 0.32 |
| 3 E2 Dynal COOH pH 5.0 | 1.574 | 0.635 | 0.024 | 0.15 |
| 3 E2 MagPrep pH 5.0 | 1.815 | 0.551 | 0.101 | 0.65 |
| 3 E2 micromer pH 5.0 | 1.794 | 0.557 | 0.061 | 0.48 |
| 3 E2 micromer pH 6.1 | 1.819 | 0.550 | 0.082 | 0.50 |

It can be seen from table 1 and figure 3 that it is possible to isolate DNA from a solution comprising said DNA using different kinds of commercially available carboxy-functionalized beads. Recovery of DNA is possible using any combination of binding ligand and carboxy-functionalized beads tested in this example (Table 1), however, it can be seen that the optimum binding ligand may vary depending on the kind of solid phase employed. Similarly, the optimum pH for binding may also vary depending on the kind of carboxy-functionalized beads and/or binding ligand employed. For example, by using Micromod Micromer-M COOH beads at a pH of 6.1 and polyethylene imine (1 E1) as a binding ligand only about 1.63 µg DNA were recovered from a solution comprising about 5 µg of said DNA. On the other hand, using spermine (3 E1) 4.78 µg in combination with Mircomod Mircomer-M COOH beads at a pH of 6, e.g. more than 95% of the initial DNA are recovered from the solution at the same pH (see figure 3).

### Example 4:

### Binding to carboxy-functionalized beads in the presence and in the absence, respectively, of a binding ligand

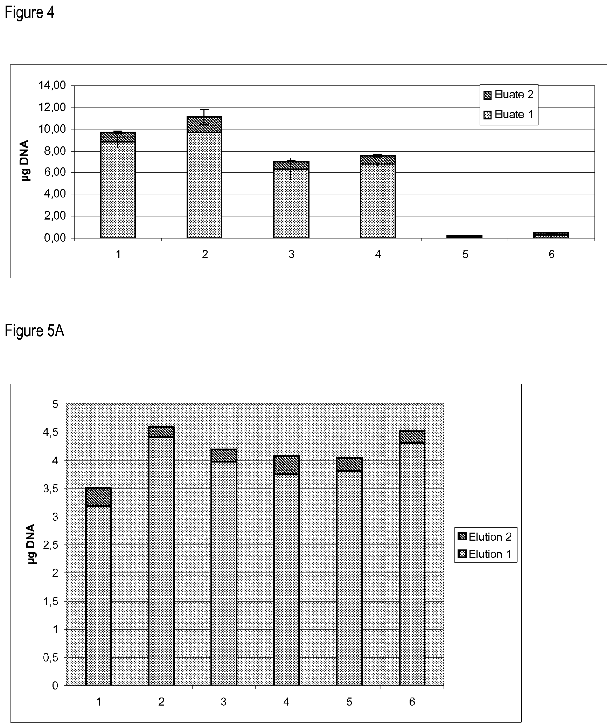

Seradyn MGCM beads were vortexed and transferred into the wells of a multiwell plate, using 3 mg/well. The beads were magnetically separated from the storage buffer and the storage buffer was removed. 20 µL of the mixture of plasmid DNA (10 µg pUC21/well, c = 1.1 µg/µL) and 10 µL of an amine solution (c = 1 µg/µL in water, pH 6.0), either comprising polyethylene imine (PEI) (Mn ≈ 423) or pentaethylene hexamine, respectively, were added to the beads. For mixtures not comprising a binding ligand (columns 5 and 6 in Fig. 4) a mixture of DNA and water instead of an amine solution was used. 100 µL of a binding buffer comprising 50 mM MES at pH 5.0 or 5.5, respectively, were added to the beads. The beads were mixed by manually pipetting up and down and shaking the multiwell plate at 1,000 rpm at room temperature for 5 min on a laboratory shaker. The beads were magnetically separated and the "flow-through" was discarded. The beads were washed twice with 100 µL water each, manually resuspended and shaken on a laboratory shaker at 1,000 rpm for 5 min at room temperature. The nucleic acid were eluted from the beads using 50 µL of the elution buffer described in example 3. The eluates of the first elution step were collected and the elution was repeated. The eluates of the first and the second elution step were collected separately and analyzed at a Nanodrop by measuring the absorbance at a wavelength of 260, 280, and 320 nm, respectively, using the elution buffer as a blank. The results are presented in table 2 and figure 4.

**Table 2:**

| Sample | 260/280 | 260/320 | total DNA [µg] | average [µg] |
|---|---|---|---|---|
| E1 PEI pH 5.5 | 1.87 | 1.83 | 8.51 | 8.86 |
| E1 PEI pH 5.5 | 1.88 | 1.86 | 9.21 | |
| E1 PEI pH 5.0 | 1.87 | 1.59 | 9.67 | 9.67 |
| E1 pentaethylene hexamine pH 5.5 | 1.88 | 1.59 | 5.63 | |
| E1 pentaethylene hexamine pH 5.5 | 1.88 | 1.73 | 7.02 | 6.32 |
| E1 pentaethylene hexamine pH 5.0 | 1.87 | 1.67 | 6.72 | |
| E1 pentaethylene hexamine pH 5.0 | 1.87 | 1.67 | 6.88 | 6.80 |
| Blank | 0.64 | 0.26 | | |
| E2 PEI pH 5.5 | 2.05 | 1.48 | 0.79 | |
| E2 PEI pH 5.5 | 1.93 | 1.57 | 0.92 | 0.85 |
| E2 PEI pH5.0 | 1.86 | 1.74 | 1.96 | |
| E2 PEI pH 5.0 | 1.93 | 1.43 | 1.02 | 1.49 |
| E2 pentaethylene hexamine pH 5.5 | 2 | 1.25 | 0.66 | |
| E2 pentaethylene hexamine pH 5.5 | 1.97 | 1.41 | 0.78 | 0.72 |
| E2 pentaethylene hexamine pH 5.0 | 1.97 | 1.35 | 0.83 | |
| E2 pentaethylene hexamine pH 5.0 | 1.99 | 1.38 | 0.73 | 0.78 |
| Blank | -4.4 | -0.71 | | |
| E1 without binding ligand pH5.5 | 6.12 | 0.15 | 0.10 | |
| E1 without binding ligand pH5.5 | 2.06 | 0.15 | 0.10 | 0.10 |
| E1 without binding ligand pH5.0 | 1.8 | 0.34 | 0.42 | |
| E1 without binding ligand pH5.0 | 15.54 | 0.15 | 0.16 | 0.29 |
| E2 without binding ligand pH5.5 | -2.26 | 0.14 | 0.04 | |
| E2 without binding ligand pH5.5 | 3.24 | 0.23 | 0.06 | 0.05 |
| E1 without binding ligand pH5.0 | 1.84 | 0.38 | 0.15 | |
| E1 without binding ligand pH5.0 | 1.67 | 0.42 | 0.18 | 0.16 |

In table 2 E1 denotes the eluates obtained from the first elution step, while E2 refers to the eluates obtained in the second elution step. As can be seen from table 2 and figure 4, the presence of binding ligands is necessary at both pH values employed, since in the absence of either polyethylene imine or pentaethylene hexamine no significant amount of DNA is bound to the solid phase.

In Fig. 4, the results obtained using PEI at pH 5.5 (column 1) and pH 5.0 (column 2) and pentaethylene hexamine at pH 5.5 (column 3) and pH 5.0 (column 4), respectively, are presented. For comparison, the results obtained in the absence of a binding ligand at pH 5.5 (column 5) and pH 5.0 (column 6) are shown as well.

### Example 5: Purification of plasmid DNA by elution at elevated temperatures and subsequent PCR

Seradyn MGCM beads were vortexed and added into 1.5 mL Eppendorf tubes
(1 mg per tube). The beads were separated magnetically from the storage buffer and the storage buffer was removed. 100 µL of a binding buffer comprising 50 mM MES at pH 5.0 as well as 5 µL pUC21 (5 µg) and 5 µL of the polyethylene imine solution (5 µg) already described in example 2 were added to the beads. The mixture was mixed by manually pipetting up and down and shaking as described in example 2. The beads were then washed twice using 100 µL water in each washing step, manually re-suspended and shaken at 1,000 rpm for 5 min at room temperature. The nucleic acids were eluted from the beads using 500 µL of a borate buffer (50 mM borate, pH 8.5, 50 mM NaCl) or a TRIS buffer (50 mM TRIS, pH 8.5, 50 mM NaCl), respectively, at 60,75 or 90 °C, respectively, using a block heater (thermoblock). The elution step was repeated, and the eluates of each elution step were collected separately. The eluates were analysed on a Nanodrop, using the respective elution buffer as a blank and 2 µL of eluates as the sample. The results are shown in figure 5A. In columns 1-3 the eluates obtained by eluting a borate-based elution buffer and in columns 4-6 an TRIS-based elution buffer at 60 °C (columns 1 and 4), 75 °C (columns 2 and 4), and 90 °C (columns 3 and 6), respectively, are shown.

It can be seen that most of the DNA is already eluted in the first elution step.

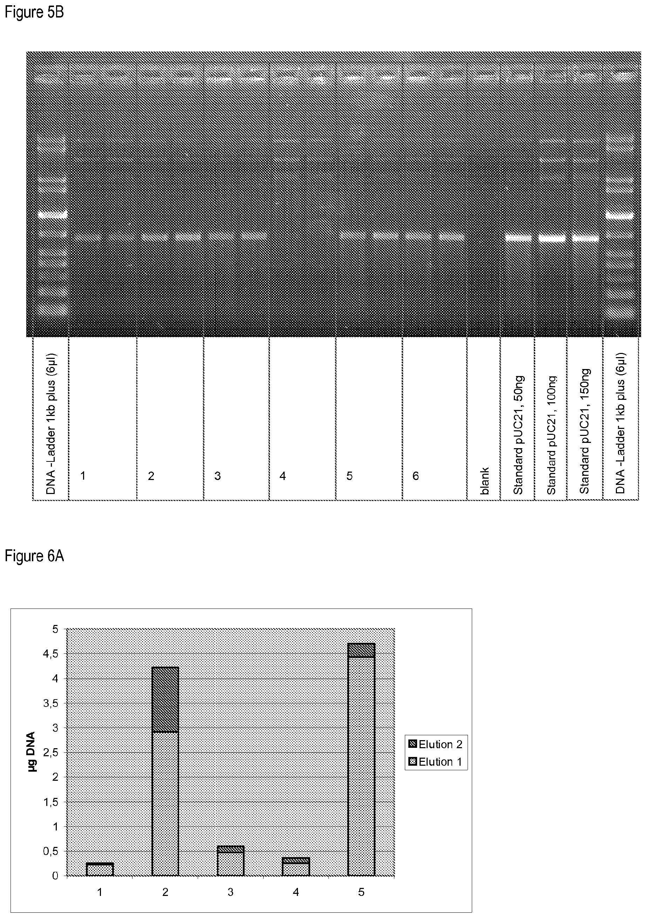

The eluates were further analysed in a PCR: For PCR amplification of the nucleic acid in the eluate, 1 µL of the eluates were mixed with 1.3 µL RNase-free water. As a positive control the standard pUC21 in different concentrations was used, as a negative control 2.3 µL RNase-free water. 50 µL of a mastermix comprising Taq PCR Mastermix (QIAGEN, Hilden, Germany) and 2 µL of each, an upstream and a downstream primer annealing to the plasmid, both commercially available from Biolegio, Nijmegen, The Netherlands, resulting in a main amplification product of a 658 bp fragment, an ampicilline-resistance gen, were mixed. 2.7 µL of this mixture was added to each eluate as well as to the positive and negative control, respectively. 5 µL of the solutions obtained from the PCR were analysed on an 1 % agarose gel. The results are presented in figure 5B.

It can be seen that excellent results were obtained for the elution at elevated temperatures. The elevated temperature do essentially not give rise to fragmentation of DNA under the conditions employed. If a temperature of 75 °C or above is employed for eluting, no inhibition of the subsequent PCR is observed even if a (nitrogen-containing) TRIS elution buffer is used.

### Example 6: Purification of plasmid DNA using different binding ligands and subsequent PCR

Seradyn MGCM beads were vortexed and transferred into the wells of a 96-well multiwell plate (1 mg/well). The beads were separated magnetically from the storage buffer and the storage buffer was removed. 100 µL of a binding buffer comprising 50 mM MES (pH 5.0), as well as 5 µg pUC21 and 5 µL of a binding solution either comprising 1: L-arginine monohydrochloride, 2: polydiallyldimethylammoniumchloride (poly-DADMAC), 3: bishexamethylenetriamine, 4: chitosane, or 5: spermine, each at a concentration of 1 mg/mL and pH 6 were added to each well. The mixtures were pipetted manually up and down and shaken at 1,000 rpm for 5 min at room temperature on a laboratory shaker. The beads were washed twice using 100 µL of water for each washing step, manually resuspended and shaken at 1,000 rpm for 5 min at room temperature. The nucleic acids were eluted from the beads by adding 15 µL of an elution buffer comprising 50 mM borate, pH 8.5 and 50 mM NaCl, resuspending the beads manually and shaking at 1,000 rpm for 5 min at room temperature. After magnetically separating the beads, the eluate was collected, and the elution step was repeated. 2 µL of each eluate was analysed at a Nanodrop using the elution buffer as a blank.

The results are shown in figure 6A. It can be seen that when employing the essentially same conditions the amount of DNA recovered from the aqueous phase varies with the binding ligand for a particular nucleic acid. In this example, the best results were obtained for plasmid DNA using poly-DADMAC and spermine, respectively. When using poly-DADMAC a second eluting step clearly is advantageous, while by using spermine most of the DNA is already eluted in the first eluting step.

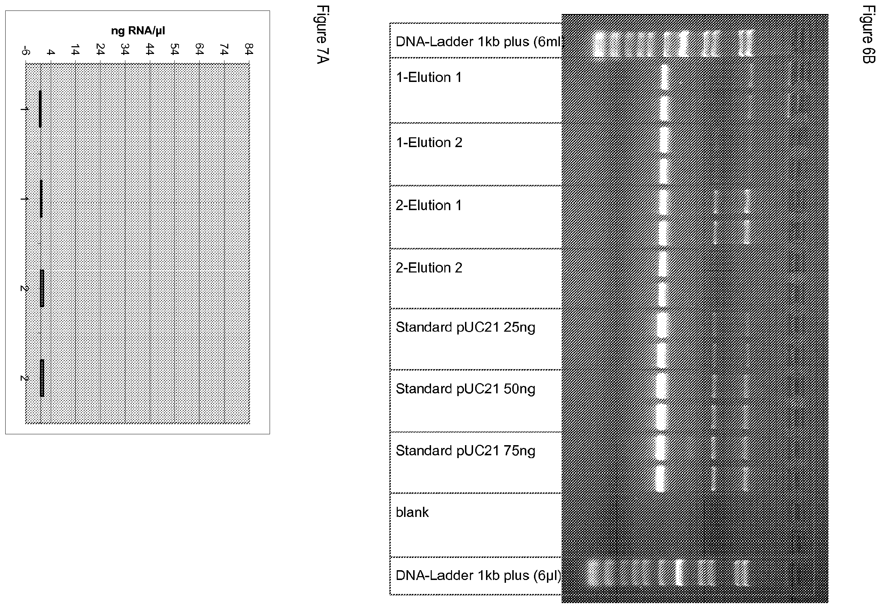

The nucleic acids in the two eluates of poly-DADMAC and spermine, respectively, were amplified in a PCR as described above and analysed on an agarose gel. The results are presented in Figure 6B (E1 = eluate of the first elution step; E2 = eluate of the second elution step).

### Example 7: Purification of RNA

Seradyn MGCM beads were vortexed and transferred into the wells of a multiwell plate using 2 mg/well. The beads were magnetically separated from the storage buffer and the storage buffer was removed. 12.5 µL of a RNA/amine solution, comprising 2.5 µL of an 16S23S rRNA solution (c = 4 µg/µL) and 10 µL of either spermine or pentaethylene hexamine, respectively, both at a concentration of 1 mg/mL in RNase-free water at a pH of about 6.107.5 µL of a binding buffer comprising 50 mM MES (pH 5.0) were added to the beads. The mixture was manually mixed by pipetting up and down and shaking the multiwell plate at 1.000 rpm for 5 min at room temperature on a laboratory shaker. The beads were magnetically separated, and the RNA content in the "flow-through" was determined at a Nanodrop, using a mixture of 107.5 µL of the above binding buffer and 10 µL of the respective amine as a blank (Figure 7A); 1: pentaethylene hexamine; 2: spermine.

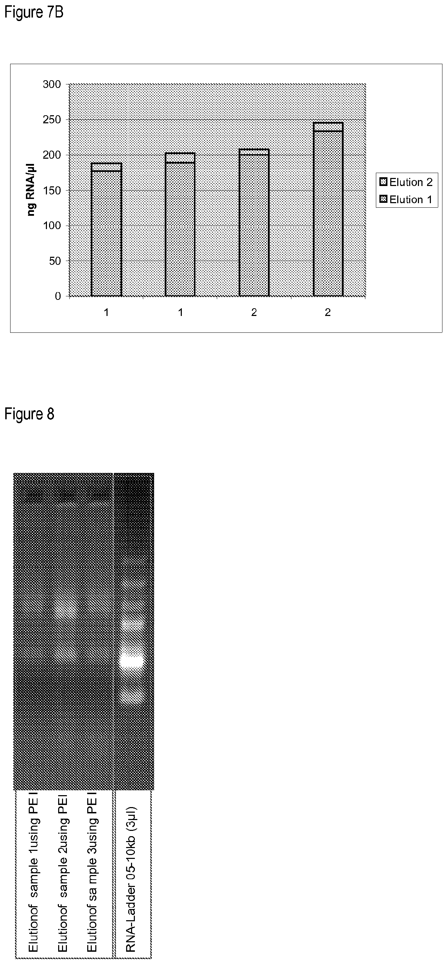

The beads were washed twice with 100 µL RNase-free water in each washing step, manually re-suspended and shaken at 1,000 rpm for 5 min at room temperature. The nucleic acids were eluted from the beads by adding 50 µL of an elution buffer comprising 50 mM TRIS and 50 mM NaCl (pH 8.5), manually re-suspending the beads and shaking the multiwell plate at 1,000 rpm for 5 min at room temperature on a laboratory shaker. The beads were magnetically separated from the solution elutate), the eluate was collected, and elution was repeated. The eluates obtained from the first and second elution step, respectively, were analysed at a Nanodrop separately, using the elution buffer as a blank (Figure 7B);1: pentaethylene hexamine; 2: spermine.

It can be seen from Figures 7A and 7B, that by using the method of the present invention, not only DNA but also RNA can be bound almost quantitatively to a solid phase.

### Example 8: Purification of rRNA from Jurkat cells

A lysis mix was prepared by mixing 3.5 mL of a surfactant-comprising lysis buffer, 35 µL Proteinase K (activity >600 mAU/mL) and 35 µL of 0.5 M DTT solution. 500 µL of this mix was added to a pellet of Jurkat cells (1x 10⁶ cells per sample). The cells were resuspended by pipetting the mixture up and down, and the mixture was then then incubated for 15 min at 60 °C. Thereafter, the mixture was cooled for 1 min on ice.

Seradyn MGCM beads (2 mg/sample ≙ 29.9 µL), 70.1 µL RNase-free water and 100 µL of the respective amine were added. As an amine, spermine (15 mM at pH 5.9 in RNase-free water) or polyethylene imine (PEI; 15 mM, pH 4.4 in RNase-free water (Mₙ≈423)) was used. The samples were mixed by pipetting up and down 5 times and incubated for 5 min at room temperature. The beads were separated from the samples magnetically, any suspended matter was allowed to settle and the clear supernatant was removed from the beads. The beads were washed with 500 µL of RNase-free water by pipetting the mixture up and down. The beads were separated magnetically and the supernatant was removed. 200 µL of a DNase digestion buffer were added (10.80 U DNase I) and the samples were mixed by pipetting up and down. The mixtures were incubated for 15 min at room temperature, the beads were separated magnetically, and the supernatant was discarded. The beads were washed again using 500 µL of RNase-free water. The nucleic acids were then eluted from the beads using 100 µL of an elution buffer, comprising 50 mM borate and 50 mM NaCl at pH of 8.5 in RNase-free water by pipetting the mixture up and down 10 times and incubating for 5 min at room temperature. The beads were then separated magnetically and the eluate was carefully collected. The amount of RNA in the eluate was determined at the Nanodrop. The amount of RNA isolated using spermine at pH 5.9 as a binding ligand was determined to be 1.48 µg (mean value of triple determination), while using polyethylene imine at pH 4.4 gave an amount of 6.12 µg RNA (mean value of triple determination).

The three eluates obtained using polyethylene imine (PEI) as a binding ligand were further analysed on a 1% formaldehyde gel, which is shown in figure 8. As a control, in lane "RNA-Ladder" a mixture of 3 µL of a commercially available 0.5-10 kb RNA Ladder (Invitrogen California, USA) and 17 µL Rnase-free water was applied. As can be seen from this gel, no significant degradation or digestion the RNA is observed, since the RNA is protected during the purification procedure by complex formation with the binding ligand.

## Claims

1. Method for isolating and/or purifying one or more nucleic acid(s) from a sample, comprising the steps of:
1) separating the nucleic acid(s) from the sample by binding the nucleic acid(s) to a solid phase by means of a binding ligand at a first pH (pH I), and
2) eluting the nucleic acid(s) from the solid phase at a second pH (pH II),
wherein the binding ligand is a non-chaotropic water-soluble cationic compound comprising at least one basic moiety and/or at least one quaternary ammonium moiety, said binding ligand forms a complex with the nucleic acid(s) at a pH below or equal to pH I and wherein the solid phase and the binding ligand are brought into contact upon or after contacting the nucleic acid(s) with the binding ligand, but not before, and wherein said first pH is lower than said second pH (pH I < pH II) and said second pH is lower than the logarithmic acidity constant pKa of the conjugated acid of said basic moiety of the binding ligand (pH II < pKa), if the binding ligand comprises one or more basic moieties or said second pH is above seven (pH 7 < pH II), if the binding ligand comprises quaternary ammonium groups, or if the binding ligand comprises both, at least one basic moiety and at least one quarternary ammonium moiety, said second pH is below the pKa of the at least one basic moiety but above pH 7.

2. A method according to claim 1, wherein the logarithmic acidity constant pKa ranges from 9 to 12, preferably from 10 to 12.

3. A method according to any of claims 1 or 2, wherein the binding ligand comprises more than one, preferably more than two and most preferably more than three basic moieties per molecule and said basic moieties preferably represents primary, secondary, and/or tertiary amino groups.

4. A method according to any of claims 1 to 3, wherein the binding ligand is a primary, secondary or tertiary mono- or polyamine.

5. A method according to claim 4, wherein the binding ligand is selected from the group comprising linear and branched alkylamines, cyclic amines, aromatic amines, heteroaromatic amines, and most preferably from the group comprising polyamines of the general structure R¹R²N[(CH₂)ₓ₁₋ₓ₇NR³]_{y}R⁴, wherein R¹, R², and R³, and R⁴ independently are selected from the group comprising hydrogen, and linear or branched C₁-C₁₈ alkyl groups, x1-x7 independently represent an integer from 2 to 8, and y ranges of from 1 to 7, preferably pentaethylene hexamine, spermidine or spermine, polylysines, polyarginines, protamines, linear and branched polyethylene imines, polyallylamine hydrochlorides, polyvinylamine hydrochlorides, polydiallylmethylamine hydrochlorides, poly(diallyldimethylammoniumchlorides) (PolyDADMAC), and poly(dimethyl
epichlorohydrine-co-ethylenediamines).

6. A method according to any of claims 1 to 5, wherein the solid phase comprises a carrier material, preferably selected from the group comprising organic polymers, polysaccharides, and inorganic carriers, more preferably selected from the group comprising polystyrenes, polyacrylates, polymethacrylates, polyurethanes, nylon, polyethylene, polypropylene, polybutylidene, and their copolymers, agarose, cellulose, dextrane, sephadex, sephacryl, chitosan, silica, quartz, glass, metal oxides, and metal surfaces.

7. A method according to any of claims 1 to 6, wherein the solid phase is in the form of (a) particle(s), including magnetic particles, (a) bead(s), including magnetic beads, a surface coating, a tube, a paper, a multiwell plate, a chip, a micro array, a tip, a dipstick, a rod, a filter plug or pad, a resin, including resins for column and spin chromatography, a mesh and/or a membrane.

8. A method according to any of claims 1 to 7, wherein the solid phase comprises acidic moieties on at least a part of that surfaces which come into contact with the sample and/or the binding ligand, preferably selected from the group comprising carboxylic (-CO2H), sulphonic (-SO3H), phosphinic (- PO2H), phosphonic (-PO3H) groups, acidic hydroxyl groups (-OH), including hydroxyl groups present at silica surfaces (silanol groups) or metal oxide species, including natural occurring minerals such as hydroxyapatite, comprising surface-exposed hydroxyl groups M-OH, wherein M may, for example, represent aluminum, calcium, titanium, zirconium, iron, or mixtures thereof.

9. A method according to any of claims 1 to 8, wherein the ratio of binding ligand to nucleic acid is equal to or above 0.5 : 1 , preferably in the range of from 0.5 : 1 to 10 : 1 (w/w), more preferably the range of from 0.5 : 1 to 2 : 1 , and most preferably is 1 : 1.

10. A method according to any of claims 1 to 9, wherein the step of binding the nucleic acid to the solid phase according to step 1) is carried out at a pH of from 1 to 8, preferably of from 2 to 7, more preferably of from 3 to 6, and most preferably of from 5 to 6.

11. A method according to any of claims 1 to 10, wherein the step of eluting the nucleic acids from the solid phase according to step 2) is carried at a pH in the range of from 7.5 to 11 , preferably of from 7.5 to 10, and most preferably of from 7.5 to 9.

12. A method according to any of claims 1 to 11, wherein the solid phase is washed at least once using an aqueous washing liquid / washing buffer comprising a salt concentration of less than 0.5 M, preferably of less than 0.2 M, more preferably of less than 0.1 M, even more preferably of less than 50 mM, and most preferably of less than 25 mM after binding the nucleic acid to the solid phase.

13. A method according to any of claims 1-11 , wherein the solid phase is washed at least once using pure water.

14. A method according to any of claims 1-13, wherein the solid phase is washed at least once using a washing liquid /washing buffer containing alcohol in a concentration of 5 % (v/v) or lower, more preferred 3 % (v/v) or lower, even more preferred 1 % (v/v) or lower, more preferred 0,5 % or lower and most preferred it does not contain any alcohol (=0 % (v/v)), and in particular it does not contain ethanol and/or propanol, especially not isopropanol.

## Patentansprüche

1. Verfahren zur Isolierung und/oder Reinigung einer oder mehrerer Nukleinsäure(n) aus einer Probe, umfassend die Schritte:
1) Abtrennen der Nukleinsäure(n) aus der Probe durch Binden der Nukleinsäure(n) an eine feste Phase mit Hilfe eines Bindungsliganden bei einem ersten pH (pH I) und
2) Eluieren der Nukleinsäure(n) von der festen Phase bei einem zweiten pH (pH II), wobei der Bindungsligand eine nicht-chaotrope wasserlösliche kationische Verbindung ist, die wenigstens einen basischen Anteil und/oder wenigstens einen quartären Ammoniumanteil umfasst, wobei dieser Bindungsligand einen Komplex mit der/den Nukleinsäure(n) bei einem pH unterhalb oder gleich dem pH I bildet und wobei die feste Phase und der Bindungsligand in Kontakt gebracht werden, während oder nachdem die Nukleinsäure(n) mit dem Bindungsliganden in Kontakt gebracht wird/werden, nicht jedoch zuvor, und wobei der erste pH niedriger als der zweite pH ist (pH I < pH II) und der zweite pH niedriger als die logarithmische Säurekonstante pKa der konjugierten Säure des basischen Anteils des Bindungsliganden ist (pH II < pKa), wenn der Bindungsligand eine oder mehrere basische Anteile umfasst, oder der zweite pH oberhalb von 7 ist (pH 7 < pH II), wenn der Bindungsligand quartäre Ammoniumgruppen umfasst, oder, wenn der Bindungsligand beides enthält, wenigstens einen basischen Anteil und wenigstens einen quartären Ammoniumanteil, der zweite pH unterhalb der pKa des wenigstens einen basischen Anteil, jedoch oberhalb von pH 7 liegt.

2. Ein Verfahren gemäß Anspruch 1, wobei die logarithmische Säurekonstante pKa von 9 bis 12, bevorzugt von 10 bis 12 reicht.

3. Ein Verfahren gemäß einem der Ansprüche 1 oder 2, wobei der Bindungsligand mehr als einen, bevorzugt mehr als zwei und am meisten bevorzugt mehr als drei basische Einheiten pro Molekül umfasst und diese basischen Einheiten bevorzugt primäre, sekundäre und/oder tertiäre Aminogruppen darstellen.

4. Ein Verfahren gemäß einem der Ansprüche 1 bis 3, wobei der Bindungsligand ein primäres, sekundäres oder tertiäres Mono- oder Polyamin ist.

5. Ein Verfahren gemäß Anspruch 4, wobei der Bindungsligand ausgewählt ist aus der Gruppe umfassend lineare und verzweigte Alkylamine, cyclische Amine, aromatische Amine, heteroaromatische Amine, und am meisten bevorzugt aus der Gruppe umfassend Polyamine der allgemeinen Struktur R¹R²N[(CH₂)ₓ₁₋ₓ₇NR³]_{y}R⁴, wobei R¹, R² und R³ und R⁴ unabhängig voneinander ausgewählt sind aus der Gruppe umfassend Wasserstoff und lineare oder verzweigte C₁-C₁₈-Alkylgruppen, x1-x7 unabhängig voneinander eine ganze Zahl von 2 bis 8 darstellen und Y von 1 bis 7 reicht, bevorzugt Pentaethylenhexamin, Spermidin oder Spermin, Polylysine, Polyarginine, Protamine, lineare und verzweigte Polyethylenimine, Polyallylaminhydrochloride, Polyvinylaminhydrochloride, Polydiallylmethylaminhydrochloride, Poly(diallyldimethylammoniumchloride) (PolyDADMAC) und Poly(dimethylepichlorhydrin-co-ethylendiamine).

6. Ein Verfahren gemäß einem der Ansprüche 1 bis 5, wobei die feste Phase ein Trägermaterial umfasst, bevorzugt ausgewählt aus der Gruppe umfassend organische Polymere, Polysaccharide und anorganische Träger, weiter bevorzugt ausgewählt aus der Gruppe umfassend Polystyrole, Polyacrylate, Polymethacrylate, Polyurethane, Nylon, Polyethylen, Polypropylen, Polybutyliden und deren Copolymere, Agarose, Cellulose, Dextran, Sephadex, Sephacryl, Chitosan, Siliciumdioxid, Quarz, Glas, Metalloxide und Metalloberflächen.

7. Ein Verfahren gemäß einem der Ansprüche 1 bis 6, wobei die feste Phase in Form eines Partikels/Partikeln, einschließlich magnetischer Partikel, eines Kügelchens/Kügelchen, einschließlich magnetischer Kügelchen, einer Oberflächenbeschichtung, eines Röhrchens, eines Papiers, einer Multiwell-Platte, eines Chips, einer Mikroanordnung, einer Pipettenspitze, eines Eintauchstäbchens, eines Stabes, eines Filterstücks oder -pads, eines Harzes, einschließlich Harzen für die Säulen- oder Spin-Chromatographie, eines Gewebes und/oder einer Membran vorliegt.

8. Verfahren gemäß einem der Ansprüche 1 bis 7, wobei die feste Phase saure Anteile auf wenigstens einem Teil der Oberflächen umfasst, die mit der Probe und/oder dem Bindungsliganden in Kontakt kommen, bevorzugt ausgewählt aus der Gruppe umfassend Carbonsäuregruppen (-CO2H), Sulfonsäuregruppen (-SO3H), Phosphinsäuregruppen (-PO2H), Phosphonsäuregruppen (-PO3H), saure Hydroxylgruppen (-OH) einschließlich Hydroxylgruppen, die auf Siliciumdioxidaoberflächen vorkommen (Silanolgruppen), oder Metalloxidspezies, einschließlich natürlich vorkommender Mineralien, wie z. B. Hydroxyapatit, umfassend oberflächenexponierte Hydroxylgruppen M-OH, wobei M z. B. Aluminium, Calcium, Titan, Zirconium, Eisen oder Mischungen davon darstellen kann.

9. Ein Verfahren gemäß einem der Ansprüche 1 bis 8, wobei das Verhältnis des Bindungsliganden zu der Nukleinsäure gleich oder oberhalb von 0,5:1, bevorzugt im Bereich von 0,5:1 bis 10:1 (w/w), weiter bevorzugt im Bereich von 0,5:1 bis 2:1 und am meisten bevorzugt 1:1 ist.

10. Ein Verfahren gemäß einem Ansprüche 1 bis 9, wobei der Schritt des Bindens der Nukleinsäure an die feste Phase gemäß Schritt 1) bei einem pH von 1 bis 8, bevorzugt von 2 bis 7, weiter bevorzugt von 3 bis 6 und am meisten bevorzugt von 5 bis 6 durchgeführt wird.

11. Ein Verfahren gemäß einem der Ansprüche 1 bis 10, wobei der Schritt des Eluierens der Nukleinsäuren von der festen Phase gemäß Schritt 2) bei einem pH in dem Bereich von 7,5 bis 11, bevorzugt von 7,5 bis 10 und am meisten bevorzugt von 7,5 bis 9 durchgeführt wird.

12. Ein Verfahren gemäß einem der Ansprüche 1 bis 11, wobei die feste Phase wenigstens einmal unter Verwendung einer wässrigen Waschflüssigkeit/ eines wässrigen Waschpuffers enthaltend eine Salzkonzentration von kleiner als 0,5 M, bevorzugt von kleiner als 0,2 M, weiter bevorzugt von kleiner als 0,1 M, noch weiter bevorzugt von kleiner als 50 mM und am meisten bevorzugt von kleiner als 25 mM gewaschen wird, nachdem die Nukleinsäure an die feste Phase gebunden wurde.

13. Ein Verfahren gemäß einem der Ansprüche 1 bis 11, wobei die feste Phase wenigstens einmal unter Verwendung von reinem Wasser gewaschen wird.

14. Ein Verfahren gemäß einem der Ansprüche 1 bis 13, wobei die feste Phase wenigstens einmal unter Verwendung einer Waschflüssigkeit/eines Waschpuffers gewaschen wird, die/der Alkohol in einer Konzentrat von 5 % (v/v) oder darunter, weiter bevorzugt von 3 % (v/v) oder darunter, noch weiter bevorzugt von 1 % (v/v) oder darunter, weiter bevorzugt von 0,5 % oder darunter enthält und am meisten bevorzugt keinen Alkohol (=0 % (v/v)) enthält und insbesondere kein Ethanol und/oder Propanol, insbesondere kein Isopropanol enthält.

## Revendications

1. Procédé pour l'isolement et/ou la purification d'un ou de plusieurs acide(s) nucléique(s) à partir d'un échantillon, comprenant les étapes consistant à :
1) séparer l'acide (les acides) nucléique(s) de l'échantillon par fixation de l'acide (des acides) nucléique(s) à une phase solide au moyen d'un ligand de fixation à un premier pH (pH 1) et
2) éluer l'acide (les acides) nucléique(s) d'avec la phase solide à un second pH (pH II),
dans lequel le ligand de fixation est un composé cationique hydrosoluble non chaotropique comprenant au moins un fragment basique et/ou au moins un fragment ammonium quaternaire, ledit ligand de fixation forme un complexe avec l'acide (les acides) nucléique(s) à un pH inférieur ou égal au pH I et dans lequel la phase solide et le ligand de fixation sont mis en contact lors de ou après la mise en contact de l'acide (des acides) nucléique(s) avec le ligand de fixation, mais non avant, et dans lequel ledit premier pH est inférieur audit second pH (pH I < pH II) et ledit second pH est inférieur à la constante d'activité logarithmique pKa de l'acide conjugué dudit fragment basique du ligand de fixation (pH II < pKa) si le ligand de fixation comprend un ou plusieurs fragments basiques, ou ledit second pH est supérieur à sept (pH 7 < pH II) si le ligand de fixation comprend des groupes ammonium quaternaires, ou si le ligand de fixation comprend à la fois au moins un fragment basique et au moins un fragment ammonium quaternaire, ledit second pH est inférieur au pKa dudit au moins un fragment basique mais supérieur à pH 7.

2. Procédé selon la revendication 1, dans lequel la constante d'acidité logarithmique pKa est dans l'intervalle de 9 à 12, de préférence de 10 à 12.

3. Procédé selon l'une quelconque des revendications 1 et 2, dans lequel le ligand de fixation comprend plus d'un, de préférence plus de deux et de façon tout particulièrement préférée plus de trois fragments basiques par molécule et lesdits fragments basiques représentent de préférence des groupes amino primaires, secondaires et/ou tertiaires.

4. Procédé selon l'une quelconque des revendications 1 à 3, dans lequel le ligand de fixation est une mono- ou polyamine primaire, secondaire ou tertiaire.

5. Procédé selon la revendication 4, dans lequel le ligand de fixation est choisi dans l'ensemble comprenant des alkylamines linéaires et des alkylamines ramifiées, des amines cycliques, des amines aromatiques, des amines hétéroaromatiques, et de façon tout particulièrement préférée dans l'ensemble comprenant des polyamines de structure générale R¹R²N[(CH₂)ₓ₁₋ₓ₇NR³]_{y}R⁴, où R¹, R² et R³ et R⁴ sont choisis indépendamment dans l'ensemble comprenant un atome d'hydrogène et des groupes alkyle en C₁-C₁₈ linéaires ou ramifiés, x1-x7 représentent indépendamment un nombre entier valant de 2 à 8 et y est dans la plage de 1 à 7, de préférence la pentaéthylène-hexamine, la spermidine ou la spermine, les polylysines, polyarginines, protamines, polyéthylène-imines linéaires et polyéthylène-imines ramifiées, chlorhydrates de polyallylamines, chlorhydrates de polyvinylamines, chlorhydrates de polydiallylméthylamines, poly(chlorure de diallyldiméthyl-ammonium)s (polyDADMAC) et copolymères diméthylépichlorhydrine/ éthylènediamine.

6. Procédé selon l'une quelconque des revendications 1 à 5, dans lequel la phase solide comprend une matière de support, choisie de préférence dans l'ensemble comprenant des polymères organiques, des polysaccharides et des matières de support inorganiques, de façon plus particulièrement préférée choisie dans l'ensemble comprenant les polystyrènes, polyacrylates, polyméthacrylates, polyuréthanes, le Nylon, le polyéthylène, le polypropylène, le polybutadiène et leurs copolymères, l'agarose, la cellulose, le dextrane, le Sephadex, le Sephacryl, le chitosane, la silice, le quartz, le verre, les oxydes métalliques et les surfaces métalliques.

7. Procédé selon l'une quelconque des revendications 1 à 6, dans lequel la phase solide est sous la forme d'une(de) particule(s), incluant des particules magnétiques, d'une(de) bille(s), incluant des billes magnétiques, d'un tapissage de surface, d'un tube, d'un papier, d'une plaque multipuits, d'une puce, d'un microréseau, d'une pointe, d'une bandelette, d'une baguette, d'un bouchon ou tampon filtrant, d'une résine, incluant des résines pour chromatographie sur colonne ou centrifuge, d'un tamis et/ou d'une membrane.

8. Procédé selon l'une quelconque des revendications 1 à 7, dans lequel la phase solide comprend des fragments acides sur au moins une partie des surfaces qui entrent en contact avec l'échantillon et/ou le ligand de fixation, de préférence choisis dans l'ensemble comprenant des groupes carboxy (-CO₂H), sulfo (-SO₃H), phosphino (-PO₂H), phospho (-PO₃H), des groupes hydroxy acides (-OH), incluant des groupes hydroxy présents sur des surfaces de silice (groupes silanol) ou des espèces de type oxyde métallique, incluant des minéraux existant dans la nature, tels que l'hydroxyapatite, comprenant des groupes hydroxy M-OH exposés en surface, M pouvant par exemple représenter l'aluminium, le calcium, le titane, le zirconium, le fer ou des mélanges de ceux-ci.

9. Procédé selon l'une quelconque des revendications 1 à 8, dans lequel le rapport du ligand de fixation à l'acide nucléique est égal ou supérieur à 0,5 : 1, de préférence dans la plage de 0,5 : 1 à 10 : 1 (p/p), de façon plus particulièrement préférée dans la plage de 0,5 : 1 à 2 : 1, et de façon tout particulièrement préférée est 1 : 1.

10. Procédé selon l'une quelconque des revendications 1 à 9, dans lequel on effectue l'étape de fixation de l'acide nucléique à la phase solide selon l'étape 1) à un pH de 1 à 8, de préférence de 2 à 7, de façon plus particulièrement préférée de 3 à 6, et de façon tout particulièrement préférée de 5 à 6.

11. Procédé selon l'une quelconque des revendications 1 à 10, dans lequel on effectue l'étape d'élution des acides nucléiques d'avec la phase solide selon l'étape 2) à un pH dans l'intervalle de 7,5 à 11, de préférence de 7,5 à 10, et de façon tout particulièrement préférée de 7,5 à 9.

12. Procédé selon l'une quelconque des revendications 1 à 11, dans lequel on lave au moins une fois la phase solide en utilisant un tampon de lavage / liquide de lavage aqueux comprenant une concentration de sel inférieure à 0,5 M, de préférence inférieure à 0,2 M, de façon plus particulièrement préférée, inférieure à 0,1 M, de façon encore plus particulièrement préférée, inférieure à 50 mM, et de façon tout particulièrement préférée, inférieure à 25 mM, après fixation de l'acide nucléique à la phase solide.

13. Procédé selon l'une quelconque des revendications 1 à 11, dans lequel on lave au moins une fois la phase solide en utilisant de l'eau pure.

14. Procédé selon l'une quelconque des revendications 1 à 13, dans lequel dans lequel on lave au moins une fois la phase solide en utilisant un tampon de lavage / liquide de lavage contenant de l'alcool à une concentration de 5 % (v/v) ou moins, de façon plus particulièrement préférée de 3 % (v/v) ou moins, de façon encore plus particulièrement préférée de 1 % (v/v) ou moins, encore mieux de 0,5 % ou moins et de façon tout particulièrement préférée qui ne contient pas d'alcool (= 0 % (v/v)), et en particulier qui ne contient pas d'éthanol et/ou de propanol, en particulier pas d'isopropanol.
